# EUROPÄISCHE PATENTSCHRIFT

(11) **EP 2 707 454 B1**
(45) Veröffentlichungstag und Bekanntmachung des Hinweises auf die Patenterteilung: **31.12.2014**
(21) Anmeldenummer: 12716228.7
(22) Anmeldetag: 28.03.2012
(51) Int. Cl.: C09K 11/06, C07C 15/38, G01J 1/48, G01N 31/22

(54) **FLUORESZENZFARBSTOFF FÜR PH-SENSOR**
FLUORESCENT DYE FOR PH SENSOR
COLORANT FLUORESCENT POUR CAPTEUR DE MESURE DE pH

(30) Priorität: 11.05.2011 DE 102011101207
(43) Veröffentlichungstag der Anmeldung: 19.03.2014
(73) Patentinhaber: Sartorius Stedim Biotech GmbH, 37079 Göttingen (DE)
(72) Erfinder: RIECHERS, Daniel, D-93051 Regensburg (DE); WÜNN, Eberhard, 37077 Göttingen (DE)
(74) Vertreter: Stehl, Astrid
(86) Internationale Anmeldenummer: PCT/EP2012/001345
(87) Internationale Veröffentlichungsnummer: WO 2012/152355

(56) Entgegenhaltungen:
- WO-A1-2008/097747
- WO-A1-2009/078970
- WO-A2-2004/027388
- US-A1- 2003 049 714
- US-A1- 2006 083 688
- US-B2- 7 390 462

## Beschreibung

Die vorliegende Erfindung betrifft einen neuartigen Fluoreszenzfarbstoff, dessen Herstellungsverfahren sowie einen optischen pH-Sensor, umfassend diesen Fluoreszenzfarbstoff.

Die Bestimmung der H⁺-Aktivität in wässrigen Medien, d.h. die Messung des pH-Werts, anhand elektrochemischer Sensoren, insbesondere mittels pH-Glaselektroden, ist seit langem bekannt. Dennoch hat die Verwendung von Glaselektroden in mehrerer Hinsicht auch Nachteile, wie beispielsweise das Problem der Miniaturisierung. Insbesondere für biotechnologische Anwendungen, in welchen eine nicht-invasive Bestimmung des pH-Werts erforderlich ist, zeigen gewöhnliche elektrochemische Sensoren ihre Schwächen.

Deshalb wurden in den letzten Jahren viele Anstrengungen unternommen, optische pH-Sensoren zu entwickeln, die einerseits die bekannten Vorteile der pH-Glaselektroden mitbringen, andererseits auch für sensible pH-Messungen biotechnologischer Anwendungen einsetzbar sind.

Anfängliche optische pH-Sensoren zeigten jedoch u.a. das Problem des "leaching", weshalb diese Sensoren meist nur einmal verwendet werden konnten ("single-use"). Zuletzt wurden vermehrt fluoreszenzbasierte optische pH-Sensoren beschrieben, wobei ein Fluoreszenzfarbstoff als "indikator" fungiert. Solche Fluoreszenzfarbstoffe weisen in Abhängigkeit vom pH-Wert eine Änderung der Anregungs- oder Emissionswellenlänge auf. Besitzt der fluoreszierende Farbstoff beispielsweise zwei verschiedene Anregungswellenlängen, so kann aus dem Quotienten der bei beiden Anregungswellenlängen nacheinander gemessenen Intensitäten der pH-Wert bestimmt werden ("Dual-Excitation"/"Single-Emission"-Verfahren). Dieses ratiometrische Messverfahren stellt eine besonders zuverlässige Methode für die pH-Bestimmung dar, da es relativ störungsunanfällig, beispielsweise gegenüber Intensitätsschwankungen, ist.

Dieses Prinzip zur Messung des pH-Werts in wässrigen Medien für biotechnologische Anwendungen unter Verwendung von fluoreszenzbasierten optischen Chemosensoren wird beispielsweise in US 7,390,462 B2 beschrieben. Hierzu werden die Fluoreszenzfarbstoffe Hydroxypyrentrisulfonsäure (HPTS) bzw. in einer weiteren Ausführungsform Dihydroxypyrendisulfonsäure (DHPDS) in einer analytpermeablen Matrix immobilisiert und in Container, wie beispielsweise Bioreaktoren, eingebracht. Das Auslesen der Sensoren erfolgt mittels einer geeigneten Optoelektronik durch die transparente Wandung des Containers nach dem vorstehend genannten "Dual-Excitation"/"Single-Emission"-Verfahren. Für die HPTS-basierte Variante wird die Fluoreszenz des Farbstoffs bei 408 nm und bei 468 nm mittels geeigneter LEDs angeregt, und die Fluoreszenzemission bei 515 nm wird mittels einer Photodiode vermessen. Aus dem Quotienten der bei beiden Anregungswellenlängen nacheinander gemessenen Intensitäten berechnet sich der pH-Wert nach einem sigmoiden Zusammenhang.

Für die DHPDS-basierten Sensoren liegen die Maxima der Exzitation bei 404 nm und 457 nm, das Maximum der Emission bei 502 nm. Die beiden Maxima der Exzitation korrelieren für beide Farbstoffe jeweils mit dem konjugierten Säure-Base-Paar des jeweiligen Farbstoffes.

Aufgrund der Anregung beider Farbstoffe im Wellenlängenbereich bei 404 nm, respektive 408 nm, lassen sich entsprechende Optoelektroniken nicht über preisgünstige POF ("Polymeroptische Fasern"), welche beispielsweise aus Polymethylmethacrylat (PMMA) bestehen (z.B. PGR-FB2000, Firma Toray), an die Sensoren anbinden. Bei diesen niedrigen Wellenlängen fluoreszieren die PMMA-POF selbst und führen zu einer Verfälschung des Messsignals.

Der Messbereich für den HPTS- und den DHPDS-basierten Sensor wird in US 7,390,462 B2 mit ca. pH 6-9 angegeben. Dieser Messbereich ist für biotechnologische Anwendungen, hier speziell die Kultivierung von Säugetierzellen, im Allgemeinen ausreichend.

Für Insektenzellkultivierungen, wie beispielsweise die Zelllinie SF9, sowie ungeregelte Batchkultivierungen von *Escherichia coli,* sowie Kultivierungen von Hefen, wie beispielsweise *Pichia pastoris* oder *Saccharomyces cerevisiae,* ist jedoch ein sensitiver pH-Messbereich im Bereich von pH 4 bis pH 8 erforderlich. Hierzu können die in US 7,390,462 B2 beschriebenen Sensoren nicht eingesetzt werden bzw. müssten unter Verwendung von zusätzlichen Farbstoffen (Hilfsfarbstoffen), wie beispielsweise Fluoreszein oder dessen Derivate, aufwendig abgeändert werden, um diesen Messbereich abzudecken.

Zudem weisen diese HPTS- bzw. DHPDS-basierten Sensoren einen Nachteil dahingehend auf, dass die Bestimmung des pH-Werts stark von der Ionenstärke im zu messenden Medium beeinflusst wird. Dieses Problem zeigt sich insbesondere bei kontinuierlichen pH-Messungen in biotechnologischen Anwendungen, in welchen die Elektrolytkonzentration bzw. die Ionenstärke in den zu untersuchenden Proben beachtlich variieren kann. Falls der optische pH-Sensor gegenüber einer Änderung der Ionenstärke sensibel ist, besteht die Gefahr, dass die erhaltenen Messergebnisse mit Fehlern behaftet sind.

Aufgabe der vorliegenden Erfindung ist es daher, einen Fluoreszenzfarbstoff sowie einen optischen pH-Sensor, welcher diesen Fluoreszenzfarbstoff umfasst, bereitzustellen, der die vorstehend genannten Nachteile bekannter optischer pH-Sensoren nicht aufweist. Insbesondere liegt dieser Erfindung die Aufgabe zugrunde, einen Fluoreszenzfarbstoff bereitzustellen, der im Vergleich zu herkömmlichen fluoreszenzbasierten optischen pH-Sensoren einen erweiterten, insbesondere in stärker saure pH-Werte verschobenen Messbereich, ohne Verwendung zusätzlicher Hilfsfarbstoffe, ermöglicht. Zudem sollte dieser Fluoreszenzfarbstoff relativ unempfindlich gegenüber Änderungen der Ionenstärke und gleichzeitig kostengünstig produzierbar sein.

Diese Aufgabe wird durch die in den Ansprüchen gekennzeichneten Ausführungsformen gelöst.

Insbesondere wird ein Fluoreszenzfarbstoff mit der folgenden Formel (I) bereitgestellt 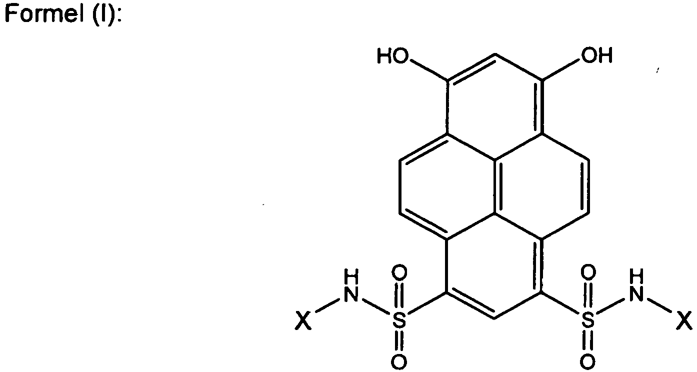 wobei X ausgewählt ist aus Wasserstoff, einer unsubstituierten oder substituierten C₁₋₂₀ Alkylgruppe, einer unsubstituierten oder substituierten C₁₋₂₀ Alkoxylgruppe, einer unsubstituierten oder substituierten C₁₋₂₀ Alkenylgruppe, einer unsubstituierten oder substituierten C₁₋₂₀ Alkinylgruppe, einer unsubstituierten oder substituierten C₁₋₂₀ Arylgruppe und einer unsubstituierten oder substituierten C₁₋₂₀ Heteroarylgruppe, wobei die Substituenten aus der Gruppe ausgewählt sind, umfassend Halogene, Hydroxyl, Carbonyl, Cyano, Thionyle, Aminogruppen, Amide und Carboxylgruppen, und deren Salze oder Kombinationen davon.

In einer bevorzugten Ausführungsform weist der erfindungsgemäße Fluoreszenzfarbstoff die folgende Formel (II) auf 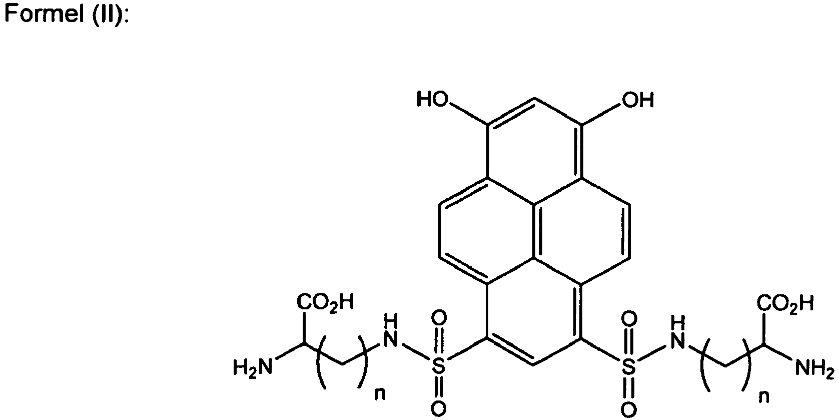 wobei n eine ganze Zahl von 1 bis 20 ist. Besonders bevorzugt ist der Fluoreszenzfarbstoff der Formel (II), wobei n eine ganze Zahl von 2 bis 4 ist. Gemäß der vorliegenden Erfindung sind die in der Formel (II) gezeigten Repetiereinheiten, welche durch n ausgedrückt sind, nicht auf lineare Kohlenstoffketten beschränkt, d.h. der erfindungsgemäße Fluoreszenzfarbstoff der Formel (II) kann in den entsprechenden Wiederholungseinheiten sowohl geradkettige Alkylgruppen als auch verzweigte Alkylgruppen aufweisen.

Gemäß dieser Ausführungsform umfasst der erfindungsgemäße Fluoreszenzfarbstoff der Formel (II), wobei n eine ganze Zahl von 2 bis 4 ist, insbesondere 1,3-Bis[*N*-(3-amino-3-carboxypropyl)sulfamoyl]-6,8-dihydroxypyren, 1,3-Bis[*N*-(3-amino-3-carboxy-1-methylpropyl)sulfamoyl]-6,8-dihydroxypyren, 1,3-Bis[*N*-(3-amino-3-carboxy-2-methylpropyl)sulfamoyl]-6,8-dihydroxypyren, 1,3-Bis[N-(4-amino-4-carboxybutyl)sulfamoyl]-6,8-dihydroxypyren, 1,3-Bis[*N*-(3-amino-3-carbo-xy-1,1-dimethylpropyl)sulfamoyl]-6,8-dihydroxypyren, 1,3-Bis[*N*-(3-amino-3-carboxy-2,2-dimethylpropyl)sulfamoyl]-6,8-dihydroxypyren, 1,3-Bis[*N*-(3-amino-3-carboxy-1,2-dimethylpropyl)sulfamoyl]-6,8-dihydroxypyren, 1,3-Bis[*N*-(3-amino-3-carboxy-1-ethyl-propyl)sulfamoyl]-6,8-dihydroxypyren, 1,3-Bis[*N*-(3-amino-3-carboxy-2-ethylpropyl)-sulfamoyl]-6,8-dihydroxypyren, 1,3-Bis[*N*-(4-amino-4-carboxy-1-methylbutyl)sulfamo-yl]-6,8-dihydroxypyren, 1,3-Bis[*N*-(4-amino-4-carboxy-2-methylbutyl)sulfamoyl]-6,8-di-hydroxypyren, 1,3-Bis[*N*-(4-amino-4-carboxy-3-methylbutyl)sulfamoyl]-6,8-dihy-droxypyren und 1,3-Bis[*N*-(5-amino-5-carboxypentyl)sulfamoyl]-6,8-dihydroxypyren.

In einer weiteren bevorzugten Ausführungsform der vorliegenden Erfindung handelt es sich bei dem erfindungsgemäßen Fluoreszenzfarbstoff der Formel (II) um 1,3-Bis[*N*-(3-amino-3-carboxypropyl)sulfamoyl]-6,8-dihydroxypyren, dargestellt durch die nachstehende Formel (IIa), 1,3-Bis[*N*-(4-amino-4-carboxybutyl)sulfamoyl]-6,8-dihydroxypyren, dargestellt durch die nachstehende Formel (IIb), und 1,3-Bis[*N*-(5-amino-5-carboxypentyl)sulfamoyl]-6,8-dihydroxypyren, dargestellt durch die nachstehende Formel (IIc).

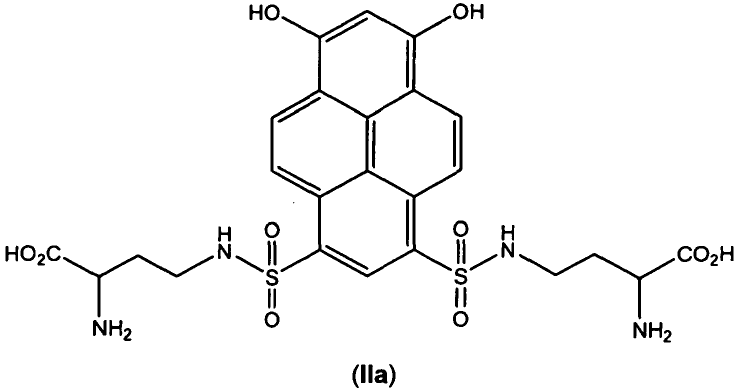

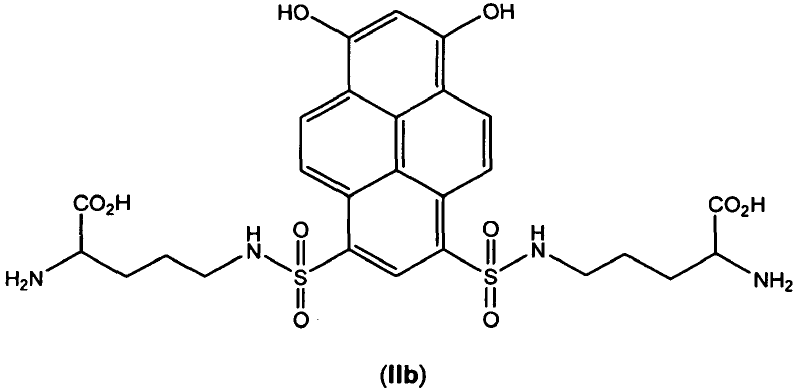

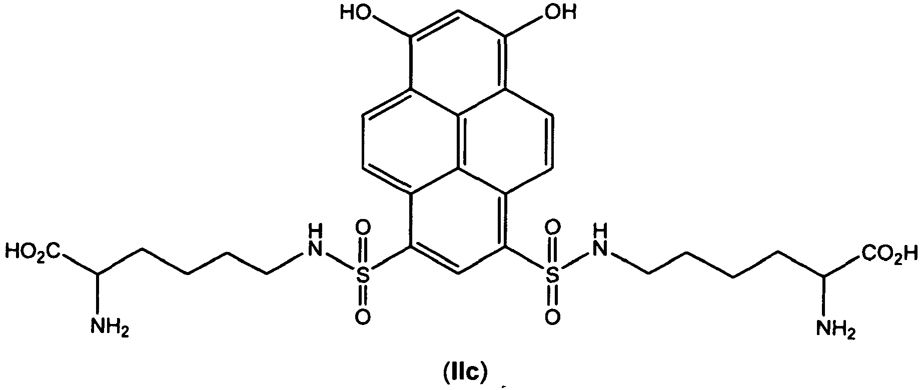

Der erfindungsgemäße Fluoreszenzfarbstoff der Formel (I) weist aufgrund seiner Pyrengrundstruktur fluoreszierende Eigenschaften auf, welche durch geeignete Wahl von X weiter verstärkt werden können. Beispielsweise kann dieser Effekt erzielt werden, wenn X Strukturmerkmale umfasst, welche über ein ausgedehntes delokalisiertes π-System verfügen, wie beispielsweise polycyclische (Hetero-)-Aromaten. In diesem Zusammenhang seien beispielsweise Naphthalin, Anthracen, Phenanthren und Tetracen erwähnt.

Erfindungsgemäß weist der Fluoreszenzfarbstoff der Formel (I) aufgrund der Hydroxylgruppen und Sulfamoylgruppen mindestens zwei verschiedene Maxima der Anregungswellenlängen oder Emissionswellenlängen, welche jeweils mit dem konjugierten Säure-Base-Paar des Fluoreszenzfarbstoffs korrespondieren, auf. Somit wird der Fluoreszenzfarbstoff der Formel (I) bei zwei verschiedenen Wellenlängen angeregt, d.h. er verfügt über zwei verschiedene Maxima in der Exzitation, oder besitzt eine Anregungswellenlänge, welche zu zwei verschiedenen Maxima der Fluoreszenzemission führt.

In einer bevorzugten Ausführungsform ist die Emission des erfindungsgemäßen Fluoreszenzfarbstoffes pH-abhängig, d.h. in Abhängigkeit des pH-Werts verändern sich die Fluoreszenzintensitäten.

Der erfindungsgemäße Fluoreszenzfarbstoff der Formel (I) weist in einer besonders bevorzugten Ausführungsform zwei verschiedene Maxima der Fluoreszenzemission auf, wovon eines dieser Maxima ein pH-abhängiges Intensitätsmaximum ist, wobei die beiden Maxima jeweils oberhalb des violetten Bereichs liegen. Insbesondere liegt das pH-abhängige Fluoreszenzmaximum vorzugsweise in einem Bereich von > 420 nm, besonders bevorzugt in einem Bereich von > 450 nm, also im blauen Bereich oder oberhalb.

Somit lassen sich vorteilhafterweise pH-abhängige Intensitätsveränderungen der Fluoreszenz des erfindungsgemäßen Fluoreszenzfarbstoffes in Bereichen verfolgen, welche nicht in den Fluoreszenzbereich fallen, welcher beispielsweise durch einen verwendeten Träger oder weitere äußere Einflüsse hervorgerufen wird. Darüber hinaus erlaubt der erfindungsgemäße Fluoreszenzfarbstoff die Verwendung von Optoelektroniken, welche über preisgünstige polymeroptische Fasern auf Polymethylmethacrylat-Basis an die Sensoren angebunden werden können, wie beispielsweise PGR-FB2000, hergestellt von der Firma Toray.

Ferner wird gemäß der vorliegenden Erfindung ein Verfahren zur Herstellung des Fluoreszenzfarbstoffes mit der Formel (I) bereitgestellt. Das erfindungsgemäße Verfahren umfasst die Schritte des:
Umsetzens einer Verbindung der folgenden Formel (III) zu einer Verbindung der folgenden Formel (IV) und
Umsetzens der Verbindung mit der Formel (IV) mit einer Verbindung der Formel X-NHR, um den Fluoreszenzfarbstoff der Formel (I) zu erhalten.

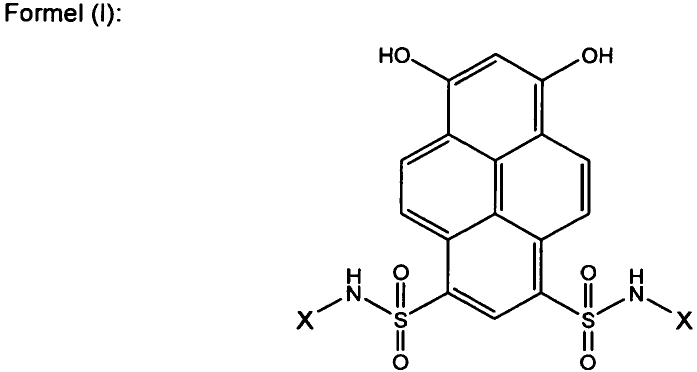

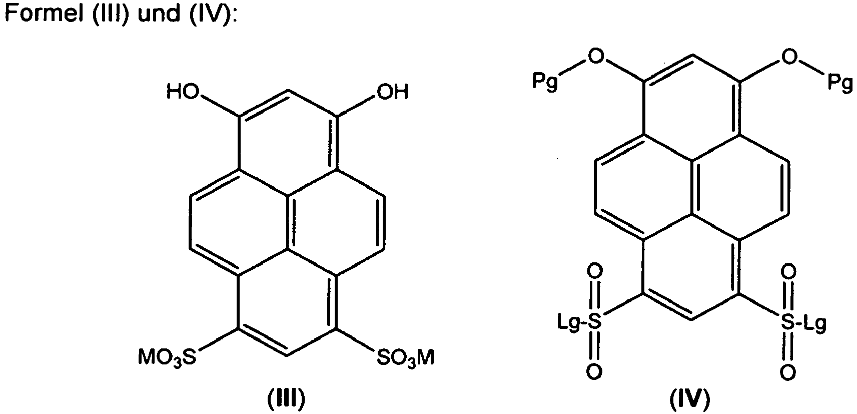

Gemäß der vorliegenden Erfindung hat X in der Formel X-NHR dieselbe Bedeutung wie vorstehend für die Formel (I) definiert.

In der Formel X-NHR stellt R Wasserstoff oder einer kationischen Gruppe dar. Die kationische Gruppe ist jede Gruppe, die mit der nachstehend definierten Lg-Gruppe eine Reaktion eingehen kann und unter Ausbildung einer Sulfonamidbindung (NH-SO₂-Bindung) abgetrennt wird. Beispielsweise seien Alkalimetallsalze erwähnt.

In der Formel (III) stellt M Wasserstoff oder ein Alkalimetall, ausgewählt aus Li, Na und K dar. Für den Fall, dass M in Formel (III) Wasserstoff ist, wird diese Verbindung im erfindungsgemäßen Verfahren vorzugsweise zunächst in ein entsprechendes Salz überführt, um die Reaktivität dieser Verbindung zu erhöhen. M stellt in der Formel (III) vorzugsweise Na dar, da dieses Na-Salz kommerziell erhältlich ist, wodurch etwaige aufwendige synthetische Vorstufen eingespart werden können.

In Formel (IV) stellt Pg eine Schutzgruppe dar. Die Schutzgruppe ist nicht eingeschränkt und kann eine dem Fachmann geläufige Schutzgruppe darstellen, die insbesondere für Hydroxylgruppen gebräuchlich ist. Beispielsweise kann die Schutzgruppe eine Silylschutzgruppe, wie beispielsweise TMS (Trimethylsilyl), TBDMS (t-Butyl-dimethylsilyl), TES (Triethylsilyl), TIPS (Triisopropylsilyl) oder TBDPS (t-Butyldiphenylsilyl), eine etherbildende Schutzgruppe, wie beispielsweise Methyl, Benzyl, p-Methoxybenzyl oder Trityl (Triphenylmethyl), eine esterbildende Schutzgruppe, wie beispielsweise Acetyl, Pivaloyl oder Benzoyl, oder eine sulfonatbildende Schutzgruppe, wie beispielsweise Tosyl (p-Toluensulfonat) oder Mesyl (Methylsulfonat), sein.

Lg stellt in Formel (IV) eine Abgangsgruppe (engl. leaving group) dar, welche aus der Gruppe, bestehend aus Triflat, Fluorosulfonat, Tosylat, Mesylat, Chlorid, Bromid und Iodid, ausgewählt ist.

Gemäß der vorliegenden Erfindung ist die Umsetzung der Verbindung (III) zu der Verbindung mit der Formel (IV) nicht eingeschränkt. Diese Umsetzung kann in einem Schritt erfolgen oder über mehrere Stufen, sogenannte Zwischenstufen, erfolgen. Beispielsweise können in dem erfindungsgemäßen Verfahren zunächst die Hydroxylfunktionalitäten in Verbindung (III) geschützt werden und anschließend die Sulfonatfunktionalität in geeigneter Weise in die Funktion -SO₂-Lg überführt werden. Das Reagenz für diesen Reaktionsschritt ist nicht begrenzt und kann jedwedes einem Fachmann bekanntes Reagenz sein, mit welchem die Abgangsgruppe Lg in die Verbindung der Formel (III) eingeführt werden kann. Beispielsweise kann Thionylchlorid für die Reaktion eingesetzt werden. In diesem Fall stellt Lg in Formel (IV) beispielsweise Chlorid dar. Der Fachmann ist zudem in der Lage, die jeweils geeignete Reaktionsbedingung, wie beispielsweise Verwendung und Wahl eines Lösungsmittels, Temperatur, Druck und Dauer, einzustellen. In der Literatur sind vergleichbare Umsetzungen beschrieben, wie beispielsweise in AT-Patent 385 755 oder F. E. Cappuccio et al., J. Fluoresc. 2004, 14, 521-533.

Gemäß der vorliegenden Erfindung umfasst das Verfahren zur Herstellung des Fluoreszenzfarbstoffes der Formel (I) den weiteren Schritt der Umsetzung der Verbindung der Formel (IV) mit einer Verbindung der Formel X-NHR. Dieser Verfahrensschritt unterliegt keiner Einschränkung. Erfindungsgemäß kann die Verbindung der Formel X-NHR ein Amin darstellen. Für diesen Fall, also dass R = H ist, kann das Amin in Abhängigkeit von der Reaktivität dieser Verbindung auch, beispielsweise *in situ,* vorab aktiviert werden.

In einer bevorzugten Ausführungsform stellt die Verbindung der Formel X-NHR ein Diamin mit einer Carboxylgruppe dar. Besonders bevorzugt ist es gemäß der vorliegenden Erfindung, dass X in dieser Formel für einen α-Aminosäurerest steht, d.h. dass diese Verbindung eine Carboxylgruppe und eine Amingruppe am selben Kohlenstoffatom aufweist. Ein Fachmann ist in solchen Fällen in der Lage, falls nötig, geeignete Schutzgruppen zu verwenden, um zu der Verbindung der Formel (I) zu kommen. Beispielsweise sei für diesen Fall, dass X in der Formel X-NHR für einen α-Aminosäurerest steht, zu erwähnen, dass die Aminfunktionalität im Rest X in geeigneter Weise geschützt wird.

Insbesondere eignen sich hierfür bekannte Schutzgruppen, wie beispielsweise Carbamate, wie Boc (t-Butylcarbamat), Fmoc (9-Fluorenylmethylcarbamat), Benzylcarbamat und Allylcarbamat.

Für den Fall, dass das Aminderivat X-NHR zunächst mit einer geeigneten Schutzgruppe versehen wurde, wird diese Schutzgruppe nach der Umsetzung dieser Verbindung mit der Verbindung der Formel (IV) wieder entfernt. Ein Fachmann ist dabei in der Lage, die Reaktionsbedingungen in Abhängigkeit von der verwendeten Schutzgruppe so zu wählen, um die Schutzgruppe wieder zu entfernen.

In einer bevorzugten Ausführungsform des erfindungsgemäßen Verfahrens zur Herstellung des Fluoreszenzfarbstoffes (I) wird als Verbindung X-NHR bei der Umsetzung mit der Verbindung der Formel (IV) eine Verbindung der folgenden Formel (V) eingesetzt: 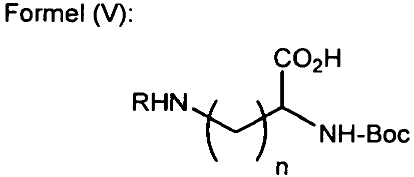 wobei R wie vorstehend definiert und n eine ganze Zahl von 1 bis 20, vorzugsweise eine ganze Zahl von 2 bis 4 ist, und die dadurch ausgedrückte Wiederholungseinheit geradkettig oder verzweigtkettig sein kann.

Ferner wird gemäß der vorliegenden Erfindung ein optischer pH-Sensor, umfassend den erfindungsgemäßen Fluoreszenzfarbstoff der Formel (I), bereitgestellt. Insbesondere umfasst der erfindungsgemäße optische pH-Sensor einen analytpermeablen Träger, auf welchem der erfindungsgemäße Fluoreszenzfarbstoff immobilisiert ist.

Gemäß der vorliegenden Erfindung wird als analytpermeabler Träger vorzugsweise eine Matrix verwendet, in welche der erfindungsgemäße Fluoreszenzfarbstoff immobilisiert werden kann. Insbesondere eignen sich als Träger nassfeste Filterpapiere oder eine ähnliche Cellulose-Matrix.

In einer bevorzugten Ausführungsform beträgt die Dicke des Trägers zwischen 50 µm und 500 µm, besonders bevorzugt zwischen 100 µm und 300 µm. Insbesondere bevorzugt sind Träger, die eine Dicke von 100 µm bis 200 µm aufweisen.

Gemäß der vorliegenden Erfindung wird der erfindungsgemäße Fluoreszenzfarbstoff der Formel (I) so auf dem Träger bereitgestellt bzw. immobilisiert, dass dieser fest mit dem Träger verbunden ist. Dadurch wird vorteilhafterweise ein sog. "leaching" des Fluoreszenzfarbstoffes vermieden. Insbesondere eignet sich für dieses Verbinden des Fluoreszenzfarbstoffes mit dem Träger ein Verfahren, in welchem der Träger mit einem Reaktivansatz, umfassend den erfindungsgemäßen Fluoreszenzfarbstoff, imprägniert wird.

Gemäß der vorliegenden Erfindung unterliegt die Herstellung des erfindungsgemäßen pH-Sensors, bzw. wie der erfindungsgemäße Fluoreszenzfarbstoff auf den Träger immobilisiert wird, keiner Einschränkung. Dabei kann der Reaktivansatz den erfindungsgemäßen Fluoreszenzfarbstoff, welcher optional zunächst aktiviert wird, und weitere Bestandteile umfassen, welche es beispielsweise erlauben, den Reaktivansatz in Form eines Hydrogels auf den Träger zu immobilisieren.

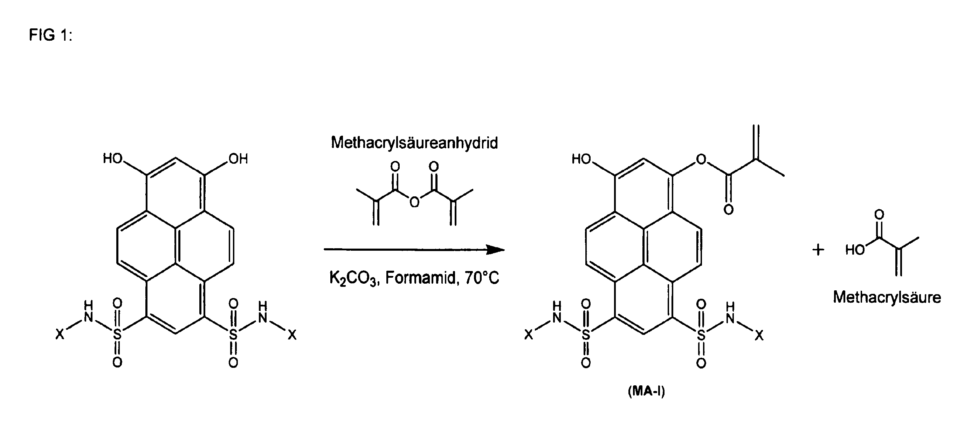

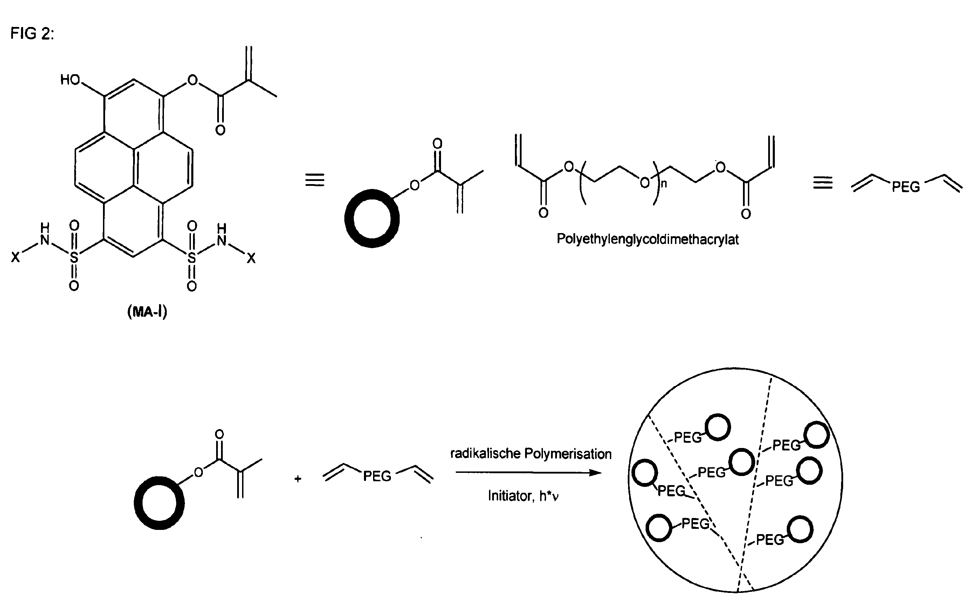

Beispielsweise kann der erfindungsgemäße Fluoreszenzfarbstoff in einem Reaktivansatz, umfassend polymerisierbare Monomereinheiten, vorliegen, mit welchem der Träger zunächst imprägniert wird, und anschließend in geeigneter Weise durch Polymerisation auf dem Träger immobilisiert werden. Als polymerisierbare Monomereinheiten eignen sich beispielsweise polymerisierbare Monomere auf (Meth)Acrylsäurebasis. Der erfindungsgemäße Fluoreszenzfarbstoff lässt sich beispielsweise analog dem in US 7,390,462 B2 beschriebenen Prozess auf dem Träger immobilisieren. Wie in Figur 1 dargestellt, kann der erfindungsgemäße Fluoreszenzfarbstoff mit Methacrylsäureanhydrid zum entsprechenden Mono-Methacrylatderivat umgesetzt werden. Dieses Mono-Methacrylatderivat des erfindungsgemäßen Fluoreszenzfarbstoffes MA-I kann beispielsweise zusammen mit Polyethylenglycoldimethacrylat (PEG-Dimethacrylat) als Reaktivansatz auf den Träger imprägniert werden und durch anschließende Polymerisation auf dem Träger immobilisiert werden (vgl. Figur 2). Insbesondere kann der Reaktivansatz neben den angesprochenen polymerisierbaren Monomereinheiten und dem Derivat des erfindungsgemäßen Fluoreszenzfarbstoffes MA-I weitere Additive umfassen, welche die Polymerisation in geeigneter Weise beeinflussen.

Insbesondere kann der Reaktivansatz ferner einen Polymerisationsinitiator umfassen, welcher thermisch und/oder photochemisch initiiert eine radikalische Polymerisation starten kann. Wie in Figur 2 gezeigt, kann der Reaktivansatz, umfassend das Derivat des erfindungsgemäßen Fluoreszenzfarbstoffes MA-I, PEG-Dimethacrylat und einen geeigneten Photoinitiator, durch Polymerisation auf dem Träger immobilisiert werden.

Beispielsweise kann die Herstellung des erfindungsgemäßen optischen pH-Sensors durch radikalische Polymerisation eines Reaktivansatzes erfolgen, welcher 1,3-Bis[*N*-(5-amino-5-carboxypentyl)sulfamoyl]-6,8-dihydroxypyren-Methacrylat, Isopropanol, 50 mM Phosphatpuffer (pH 7,0, Ionenstärke 150 mM), PEG-400-Dimethacrylat, sowie den Photoinitiator Darocur^{®} umfasst. Mit dem Reaktivansatz wird ein nassfestes Filtrierpapier der Dicke 150 µm imprägniert und je 60 sec von beiden Seiten mit UV-Licht bestrahlt (5 mW/cm², 360 nm). Die imprägnierte Matrix wird anschließend zweimal für 20 min in Reinstwasser ausgekocht, um nicht polymerisierte Bestandteile des Reaktivansatzes auszuwaschen, und anschließen bei 70°C für 24 Stunden getrocknet.

Gemäß einer bevorzugten Ausführungsform der vorliegenden Erfindung umfasst der optische pH-Sensor ferner mindestens eine Beschichtung, welche auf mindestens einer der beiden Oberflächen des Trägers, auf welchem der erfindungsgemäße Fluoreszenzfarbstoff immobilisiert ist, bereitgestellt ist. Erfindungsgemäß umfasst der pH-Sensor vorteilhafter auf beiden Oberflächen des Trägers eine Beschichtung, wodurch der pH-Sensor beispielsweise besonders wirksam vor äußeren Beschädigungen geschützt werden kann.

Diese Beschichtung kann eine dem Fachmann bekannte Beschichtung sein, welche vorzugsweise transparent ist. Insbesondere eignen sich Beschichtungen aus Glas oder Kunststoffen. Erfindungsgemäß können beispielsweise Kunststofffolien an den Träger laminiert werden. Als Beispiel sei eine PET-PE-Laminierfolie genannt, welche aus einer 40 µm dicken Polyethylenterephthalatschicht (PET) und einer 8 µm dicken Polyethylenschicht (PE) besteht. Diese Laminierfolie kann beispielsweise bei 135°C heiss anlaminiert werden.

Erfindungsgemäß ermöglicht der pH-Sensor mit einer Beschichtung zudem vorteilhaft die Bereitstellung beispielsweise einer Klebeschicht auf der Beschichtung, wodurch der erfindungsgemäße pH-Sensor beispielsweise an einem Container, in welchem das auf den pH-Wert zu prüfende bzw. zu verfolgende Medium bereitgestellt ist, fixiert werden kann. In diesem Fall lässt sich der erfindungsgemäße pH-Sensor mittels einer geeigneten Optoelektronik durch eine transparente Wandung von der Rückseite her auslesen.

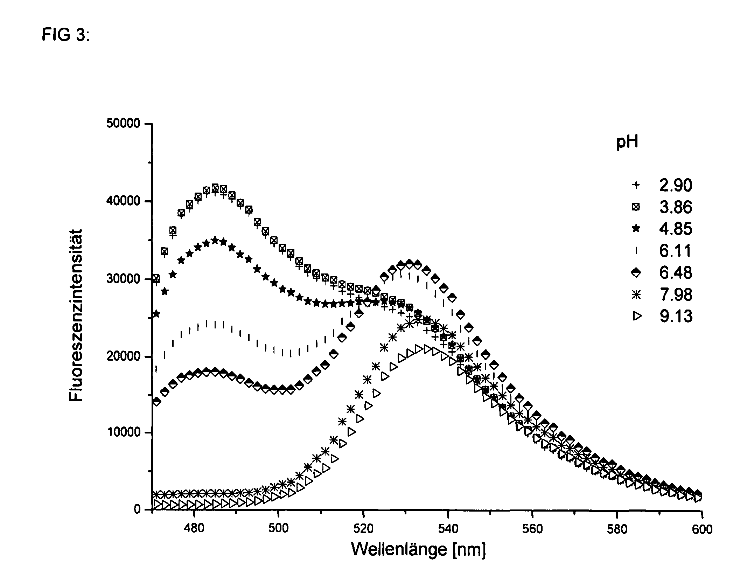

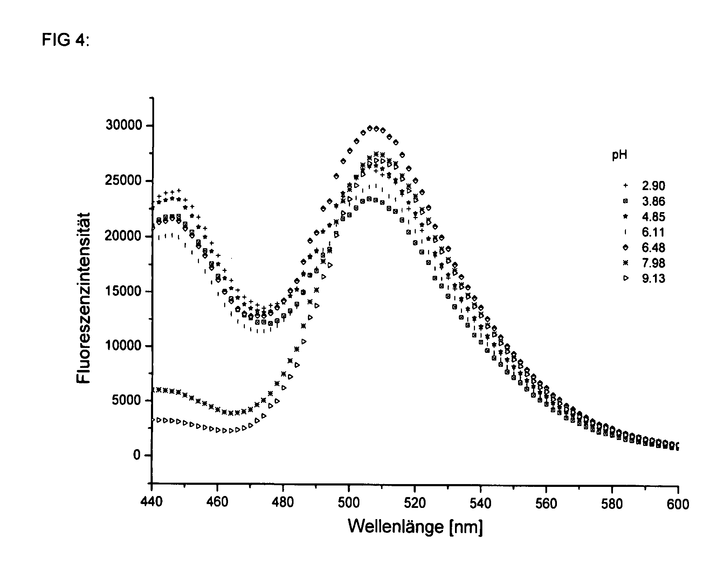

In Figur 3 sind Fluoreszenzspektren eines erfindungsgemäßen optischen pH-Sensors, umfassend 1,3-Bis[*N*-(5-amino-5-carboxypentyl)sulfamoyl]-6,8-dihydroxy-pyren (IIc) als Fluoreszenzfarbstoff, dargestellt. Die Spektren wurden mittels eines Tecan-Laborspektrofluorometers aufgenommen (Exzitation bei 445 nm). 4-mm-Rundstanzlinge des Sensormaterials wurden hierzu in einer schwarzen 96-WellPlatte (Firma Corning) mit transparentem Boden mit 50 mM-Puffern der Ionenstärke 150 mM (eingestellt mit NaCl) und pH-Werten von pH 2,9 bis pH 9,13 überschichtet und für 1 Stunde quellen gelassen. Die anschließende Messung erfolgte bei Anregung mit 445 nm (entspricht dem Maximum des Exzitationsspektrums bei Fluoreszenzmessung bei 530 nm) mit einer Auflösung von 4 nm. Das Fluoreszenzmaximum bei 485 nm korreliert mit dem pH-Wert, d.h. bei sinkendem pH-Wert detektiert man eine zunehmende Fluoreszenzintensität bei 485 nm, das zweite Maximum, welches sich pH-abhängig leicht im Bereich zwischen 535 nm und 530 nm verschiebt, zeigt keine direkte pH-Abhängigkeit und dient als spektrale Referenz für das pH-abhängige Maximum (vgl. Figur 3). Im Vergleich zeigt Figur 4 Fluoreszenzspektren eines in US 7,390,462 B2 beschriebenen und mit DHPDS hergestellten Sensors, der unter den gleichen Bedingungen getestet wurde.

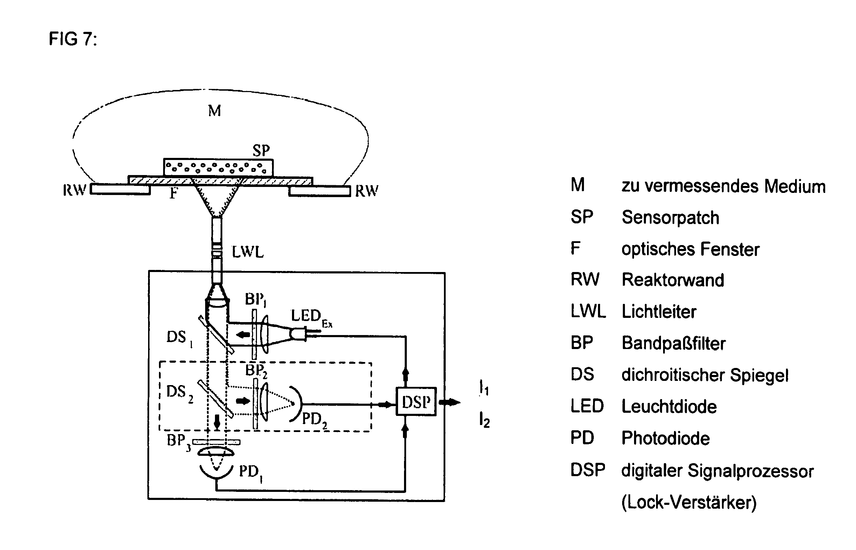

Durch Messung mittels einer geeigneten Optoelektronik, wie sie beispielsweise in Figur 7 dargestellt ist, können die Fluoreszenzintensitäten bei beiden Wellenlängen (485 nm und 530 nm) unter Exzitation mittels einer Leuchtdiode (445 nm) bestimmt werden.

Durch Anwendung eines ratiometrischen Messverfahrens, wobei die erhaltenen Intensitäten I(485 nm) und I(530 nm) in Anlehnung an das "Dual-Excitation"/"Single-Emission"-Verfahren dividiert werden, kann ein robustes und hinsichtlich Intensitätsschwankungen besonders störungsunanfälliges Messverfahren bereitgestellt werden.

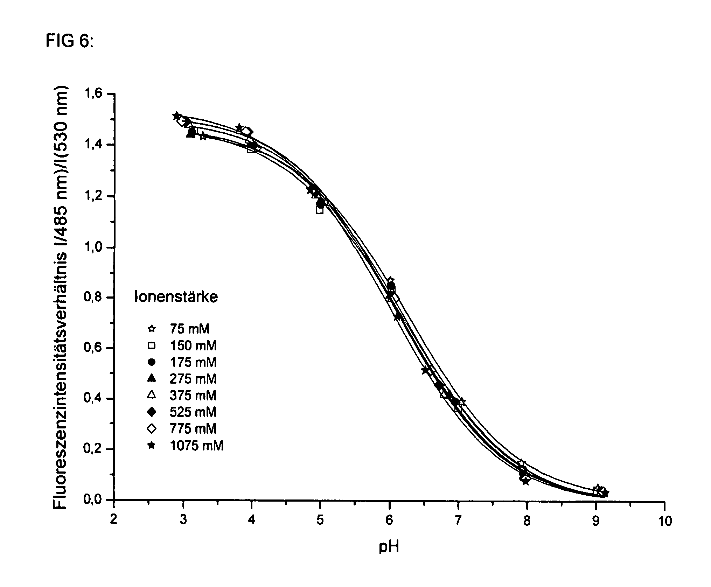

In Figur 6 sind Verläufe dargestellt, bei welchen die erhaltenen Intensitätsquotienten gegen den pH-Wert aufgetragen sind, wobei die pH-Werte im Bereich von pH 2,9 bis pH 9,1 gemessen wurden. Zudem wurden die Verläufe für verschiedene Ionenstärken der verwendeten pH-Puffer gemessen. Im getesteten Ionenstärkebereich von 75-1075 mM (mMol/L) ergibt sich für den erfindungsgemäßen optischen pH-Sensor, umfassend 1,3-Bis[*N*-(5-amino-5-carboxypentyl)sulfamoyl]-6,8-dihydroxypyren (IIc) als Fluoreszenzfarbstoff, überraschenderweise eine sehr hohe Übereinstimmung der Sensorkennlinien für alle getesteten Ionenstärken.

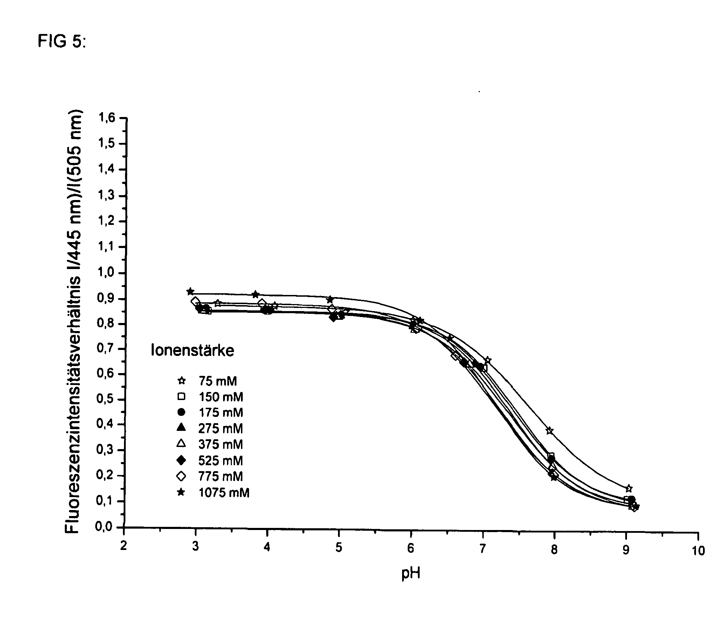

Im Vergleich zu dem unter gleichen Bedingungen getesteten, d.h. bei einer Exzitationswellenlänge von 405 nm und unter Berechnung des Intensitätsquotienten I(445 nm)/I(505 nm), in US 7,390,462 B2 beschriebenen und mit DHPDS hergestellten Sensor (vgl. Figur 5) weist der erfindungsgemäße pH-Sensor, wie in Figur 6 gezeigt, eine sehr geringe Empfindlichkeit gegenüber der Ionenstärke auf. Wie aus Figur 5 ersichtlich, verschiebt sich die Kennlinie für die in US 7,390,462 B2 beschriebenen, mit DHPDS hergestellten Sensoren mit zunehmender Ionenstärke seitwärts zu niedrigeren pH-Werten, wodurch die Genauigkeit eines derartig hergestellten und vorkalibrierten Sensors beeinträchtigt wird. Der erfindungsgemäße Sensor (vgl. Figur 6) weist eine derartige Verschiebung im Rahmen der Messgenauigkeit des Tecan-Laborspektrofluorometers nicht auf, so dass bei im Messbetrieb veränderlicher Ionenstärke präzisere Messwerte zugänglich werden. Dies ist insbesondere bei der Anwendung von vorkalibrierten optischen pH-Sensoren in geregelten Fermentationen/Zellkultivierungen von Vorteil, da durch die pH-Regelung, welche in den meisten Fällen durch Zupumpen von Base zu der sich über den Kultivierungsverlauf und den Zellmetabolismus ansäuernden Kulturbrühe erfolgt, die Ionenstärke ansteigt. Es hat sich überraschenderweise gezeigt, dass der erfindungsgemäße pH-Sensor durch diesen Ionenstärke-Anstieg nicht kompromittiert wird.

Weiterhin weist der erfindungsgemäße pH-Sensor im Gegensatz zu dem in US 7,390,462 B2 beschriebenen, mit DHPDS hergestellten Sensor einen erweiterten und in stärker saure pH-Werte verschobenen Messbereich auf. Der dynamische Messbereich des erfindungsgemäßen pH-Sensors mit dem Fluoreszenzfarbstoff IIc entspricht dem linearen Teil der Kalibrationskurve (vgl. Figur 6) und liegt hierbei zwischen pH 4,0 und pH 8,0. Aufgrund des erweiterten dynamischen Messbereichs eignet sich der erfindungsgemäße pH-Sensor auch für die Anwendung in Kultivierungen von Organismen, die in diesem Bereich ihr pH-Optimum, d.h. optimales Wachstum oder auch optimale Produktionsraten, aufweisen. Im Gegensatz dazu liegt der dynamische Messbereich des mit DHPDS hergestellten Sensors im engeren pH-Bereich von pH 6,0 bis pH 8,5.

Anhand der vorstehend aufgeführten Merkmale sowie den Messergebnissen wird deutlich, dass der erfindungsgemäße neuartige Fluoreszenzfarbstoff (I) als Indikator in pH-Sensoren überraschenderweise zu einem erweiterten, insbesondere in stärker saure pH-Werte verschobenen Messbereich führt, ohne dass zusätzliche Hilfsfarbstoffe benötigt werden. Damit stellt der erfindungsgemäße optische pH-Sensor ein effektives System dar, welches auf kostengünstige Weise produzierbar ist und über einen breiten pH-Bereich, insbesondere in biotechnologischen Anwendungen, einsetzbar ist. Darüber hinaus weist der erfindungsgemäße Fluoreszenzfarbstoff bzw. optische pH-Sensor im Vergleich zu vergleichbaren bekannten optischen pH-Sensoren eine geringe Empfindlichkeit gegenüber Änderungen der Ionenstärke im zu messenden Medium auf, wodurch die Durchführung der pH-Bestimmung signifikant erleichtert wird.

Die vorliegende Erfindung wird durch die nachfolgenden Beispiele hinsichtlich des Herstellungsverfahrens des erfindungsgemäßen Fluoreszenzfarbstoffes (I) näher erläutert, ohne darauf beschränkt zu sein.

### Beispiele

### Allgemeines:

¹H-NMR-Spektren wurden bei 300 MHz mit einem Varian Mercury Vx300 Spektrometer in CDCl₃, D₂O, [D₄]-Methanol, [D₆]-Aceton oder [D₆]-DMSO aufgenommen. CHCl₃/CDCl₃, DHO,CD₂HOD, C₃HD₅O und CD₃SOD₂H dienten als interne Referenz. ESI-MS- und HRMS-Spektren wurden mittels Finnigan-LCQrespektive Bruker-Daltonic-APEX IV-7T-FTICR-Geräten aufgenommen. Die HPLC-Analyse wurde mittels Synergi MAX-RP C12 als Festphase und MeOH/H2O + 0.05% Ameisensäure als Eluent durchgeführt. Schmelzpunkte wurden mit einem Büchi 510 Kapillarmessgerät ermittelt. Alle Chemikalien wurden im kommerziell höchsten erhältlichen Reinheitsgrad eingesetzt. Wasserfreies Dichlormethan und THF wurden mittels Molekularsieb 4 Å nach [D1] hergestellt. Wasserfreies Pentan wurde über Natrium destilliert. Alle Reaktionen wurden in Abwesenheit von Licht durchgeführt. Ansätze mit wasserfreien Solventien wurden in Argonatmosphäre in flammengetrockneten Glasgeräten gefahren.

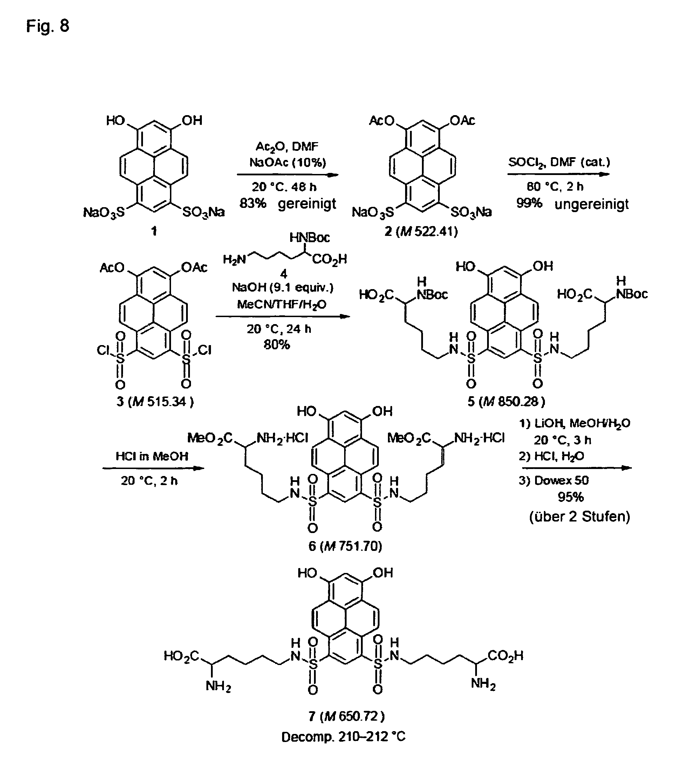

Im Folgenden wird die Synthese von 1,3-Bis[*N*-(5-amino-5-carboxypentyl)sulfamoyl]-6,8-dihydroxypyren IIc (7) nach dem in Figur 8 dargestellten Schema beschrieben.

### 6,8-Diacetoxypyren-1,3-disulfonsäurenatriumsalz (2)

Verbindung (2) wurde nach dem bereits publizierten [D2], jedoch hier abgewandelten Protokoll hergestellt. Als Abwandlung wurde Natriumacetat eingesetzt [D3]. Eine Mischung aus Natrium-6,8-Dihydroxypyren-1,3-disulfonat (1) (4,452 g, 10,16 mmol), Natriumacetat (83,0 mg, 1,016 mmol) und Essigsäureanhydrid (5,8 mL) in DMF (250 mL) wurde intensiv bei Raumtemperatur für 48 h unter Lichtausschluss gerührt. Der Reaktionsansatz wurde durch ein Celite-Kieselgur-Bett (h =0,5 cm) filtiert, DMF wurde per Rotationsverdampfer bei 50°C und 0,1 Torr abgezogen. Der Rückstand wurde aus einer Ethanol/Wasser-Mischung (70 ml:10 ml) umkristallisiert, mit Aceton gewaschen und im Vakuum zu einer Ausbeute von 4,242 g (83%) als gelbes Pulver (Schmelzpunkt 245-247°C (Zersetzung) getrocknet. ¹H NMR (300 MHz, D₂O): δ = 9,07 (s, 1 H),8,57 (d, *J* = 9,0 Hz, 2 H), 7,64 (d, *J* = 9,0 Hz, 2 H), 6,90 (s, 1 H), 2,30 (s 6 H, 2 CH₃).

### 6,8-Diacetoxypyren-1,3-disulfonyldichlorid (3)

Verbindung (3) wurde in Abwandlung des in [D3] angegebenen Protokolls dargestellt. Zum Diacetat 2 (5,080 g, 9,724 mmol) wurden 5 Tropfen DMF hinzugefügt. Zu dieser Mischung wurden vorsichtig 30 ml Thionylchlorid unter Rühren und Kühlen (Eisbad) hinzugefügt. Die Suspension wurde für 2h refluxiert, hierbei färbte sich die Suspension braun. Nach Abkühlen auf 25°C unter Argon-Atmosphäre wurde überschüssiges Thionylchlorid im Vakuum abdestilliert. Der gelbe Rückstand wurde in 50 ml wasserfreiem Dichlormethan resuspendiert und anschließend zentrifugiert. Der Überstand wurde mittels einer Spritze in einen Rundkolben überführt. Dieser Vorgang wurde weitere fünf Mal wiederholt. Die vereinigten Überstände wurden unter Argon-Atmosphäre auf 50 ml eingedampft. 200 ml wasserfreies Pentan wurde hinzugefügt, nach Lagerung über Nacht bei 4°C wurde der Überstand entfernt. Der Rückstand wurde unter reduziertem Druck zur Gewichtskonstanz zu einem gelben Feststoff getrocknet (4,962 g, 99%). ¹H NMR (300 MHz, CDCl₃): δ = 9,50 (s, 1 H), 9,18 (d, *J* = 9,0 Hz, 2 H), 8,77 (d, *J* = 9,0 Hz, 2 H), 8,14 (s, 1 H), 2,62 (s 6 H, 2 CH₃).

### 1,3-Bis{N-[5-(tert-butoxycarbonylamino)-5-carboxypentyl]sulfamoyl}-6,8-dihydroxypyren (5)

Die Herstellung von strukturisomeren Derivaten zu **(5)** wurde bereits in [D4] veröffentlicht, allerdings ohne Angaben zu den experimentellen Bedingungen für die Herstellung dieser Derivate. Unter Argonatmosphäre wurde Boc-Lys-OH **(4)** (2,159 g, 8,765 mmol) in wässriger 1N Natronlauge (40 mL) gelöst, anschließend wurde Acetonitril hinzugefügt (140 mL), die Reaktionsmischung wurde mittels Eisbad gekühlt. Unter energischem Rühren wurde eine Lösung des Dichlorids (3) (2,258 g, 4,382 mmol) in wasserfreiem THF (100 mL) tropfenweise über einen Zeitraum von 3 h bei 4°C hinzugegeben. Der Ansatz wurde weitere 21 h bei Raumtemperatur gerührt und anschließend unter reduziertem Druck aufkonzentriert. Der Rückstand wurde in Wasser aufgenommen (100 mL), so dass eine smaragdgrüne Lösung entstand. Die Lösung wurde mit 1N Salzsäure auf pH 3,5 titriert (ca. 23 ml). Der resultierende braune Niederschlag wurde sofort abzentrifugiert, mit Wasser gewaschen und über Phosphorpentoxid über Nacht zu einem braunen Pulver getrocknet (2,981 g, 80%). ¹H NMR (300 MHz, [D₆]-Aceton): δ =9,19 (s, 1 H), 8,76 (d, *J* = 9,0 Hz, 2 H), 8,66 (d, *J* = 9,0 Hz, 2 H), 7,47 (s, 1 H), 6,98 (br. s, 2H, 2 NH), 6,06 (d, *J* = 9,0 Hz, 2 H, 2 NH), 4,04 (br. s, 2 H, 2 CHN), 2,95 (q, J = 6,0 Hz, 4 H, 2 C*H*₂N), 1,72-1,40 (m, 12 H, 6 CH₂), 1,36 (s, 18 H, 2 *t*Bu). HRMS (ESI): *m*/*z* [M + Na]⁺ berechnet für C₃₈H₅₀N₄NaO₁₄S₂ 873,2657; gefunden 873,2659; berechnet für C₃₈H₅₀N₄O₁₄S₂ 850,2765; gefunden 850,2743.

### 1,3-Bis[N-(5-amino-5-methoxycarbonylpentyl)sulfamoyl]-6,8-dihydroxypyren Dihydrochlorid (6).

Zu einer Lösung des bis-Boc-Derivats **5** (2,673 g, 3,143 mmol) in Methanol (80 mL) wurden 60 ml 10 N HCl in Methanol bei 4°C hinzugegeben. Die Reaktionsmischung wurde für 10 min bei 4°C und anschließend weitere 2 h bei Raumtemperatur gerührt, dann unter reduziertem Druck zur Trockene eingedampft. Der Rückstand wurde über Nacht über Phosphorpentoxid getrocknet. ¹H NMR und HRMS (ESI) Spektren zeigten die Bildung des Dihydrochlorids des Diesters **6.** Ausbeute 2,340 g (99%). ¹H NMR (300 MHz, CD₃OD): δ = 9,10 (s, 1 H), 8,69 (d, *J* = 9,0 Hz, 2 H), 8,61 (d, *J* = 9,0 Hz, 2 H), 7,24 (s, 1 H), 3,84-3,79 (m, 2 H, 2 CHN), 2,89 (t, *J* = 9.0 Hz, 4 H, 2 C*H*₂N), 1,75-1,63 (m, 4 H, 2 CH₂), 1,47-1,25 (m, 8 H, 4 CH₂). HRMS (ESI): *m*/*z* [M + H]⁺ berechnet für C₃₀H₃₉N₄O₁₀S₂ 679,2108; gefunden wurde 679,2102.

### 1,3-Bis[N-(5-amino-5-carboxypentyl)sulfamoyl]-6,8-dihydroxypyren (7).

Zu einer Lösung des Dihydrochlorids 6 (2,443 g, 3,250 mmol) in Methanol (80 mL) wurde eine Lösung von LiOH·H₂O (1,319 g, 31,43 mmol) in Wasser (20 mL) hinzugegeben. Der Ansatz wurde für 3 h bei Raumtemperatur stark gerührt und anschließend abgedampft. Der Rückstand wurde in 80 ml Wasser aufgenommen, 4 ml konz. Salzsäure wurden hinzugegeben. Die Reaktionsmischung wurde ein weiteres Mal eingedampft und anschließend in 100 ml Wasser aufgenommen, auf eine 4 cm x 40 cm Säule, gepackt mit saurem Dowex 50WX2-200, gegeben, mit 1,5 L Wasser gewaschen und anschließend mit 1,5 L 1N Ammoniak-Lösung eluiert. Die smaragdgrüne Lösung von **7,** welche unter reduziertem Druck bei 50°C eingedampft wurde, wurde über Phosphorpentoxid über Nacht zum dunkelbraunen Pulver 7 getrocknet (2,0350 g, 96%). Das Pulver wurde in 20 ml Wasser bei 70°C für 20 min gerührt, abgekühlt und zentrifugiert. Der Bodensatz wurde in eiskaltem Wasser gerührt, abzentrifugiert und über Nacht über Phosphorpentoxid getrocknet (1,1907 9).
Schmelzpunkt: 210-212°C (Zersetzung). ¹H NMR (300 MHz, [D₆]-DMSO): δ = 8,84 (s, 1 H), 8,53 (d, *J*= 9,0 Hz, 2 H), 8,46 (d, *J* = 9,0 Hz, 2 H), 8,15 (br. s, 2 H, 2 NH), 7,27 (s, 1 H), 3,12 (m, 2 H, 2 C*H*N; überlappend mit HOD und NH₂), 2,65 (t, *J* = 7,5 Hz, 4 H, 2 C*H*₂N), 1,57-1,35 (m, 4 H, 2 CH₂), 1,30-1,00 (m, 8 H, 4 CH₂). HRMS (ESI): *m*/*z* [M + Na]⁺ berechnet für C₂₈H₃₄N₄NaO₁₀S₂ 673,1614; gefunden 673,1609; *m*/*z* [M - H]⁺ berechnet für C₂₈H₃₃N₄O₁₀S₂ 649,1638; gefunden 649,1644.

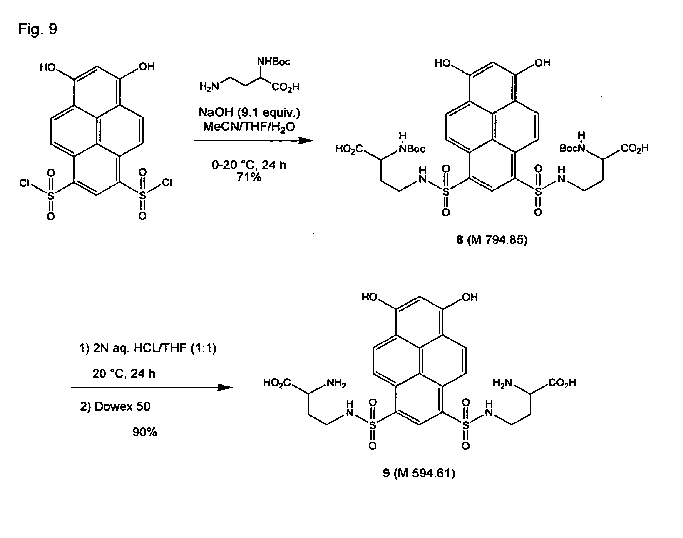

Im Folgenden wird die Synthese von 1,3-Bis[N-(3-amino-3-carboxypropyl)sulfamoyl]-6,8-dihydroxypyren (9) nach dem in Figur 9 dargestellten Schema beschrieben.

### 1,3-Bis{N-[3-tert-butoxycarbonylamino)-3-carboxypropyl]sulfamoyl}-6,8-dihydroxypyren (8)

Unter Argonatmosphäre wurde L-Boc-Dab-OH (1,288 g, 5,90 mmol) in wässriger 1N Natronlauge (30 mL) gelöst, anschließend wurde Acetonitril hinzugefügt (100 mL), die Reaktionsmischung wurde mittels Eisbad gekühlt. Unter energischem Rühren wurde eine Lösung des Dichlorids (3) (1,520 g, 2,950 mmol) in wasserfreiem THF (65 mL) tropfenweise über einen Zeitraum von 3 h bei 4°C hinzugegeben. Der Ansatz wurde weitere 21 h bei Raumtemperatur gerührt und anschließend unter reduziertem Druck aufkonzentriert. Der Rückstand wurde in Wasser aufgenommen (100 mL), so dass eine smaragdgrüne Lösung entstand. Die Lösung wurde mit 1N Salzsäure auf pH 3,5 titriert (ca. 19,5 ml). Der resultierende braune Niederschlag wurde sofort abzentrifugiert, mit kaltem Wasser (50 ml) gewaschen, nochmals zentrifugiert und über Phosphorpentoxid über Nacht zu einem braunen Pulver getrocknet (1,659 g, 71%). ¹H NMR (300 MHz, [D₆]-Aceton): δ =9,21 (s, 1 H), 8,75 (d, *J* = 9,0 Hz, 2 H), 8,67 (d, *J* = 9,0 Hz, 2 H), 7,49 (s, 1 H), 7,09 (br. s, 2H, 2 NH), 6,16 (d, *J* = 9,0 Hz, 2 H, 2 NH), 4,18 (m, 2 H, 2 CHN), 3,06 (m, 4 H, 2 C*H*₂N), 2,07-1,75 (dm, 4 H, 2 CH₂), 1,29 (s, 18 H, 2 *t*Bu). HRMS (ESI): *m*/*z* [M + Na]⁺ berechnet für C₃₄H₄₂N₄NaO₁₄S₂ 817,2037; gefunden 817,2059; berechnet für C₃₄H₄₂N₄O₁₄S₂ 794,2139; gefunden 794,2143.

### 1,3-Bis[N-(3-amino-3-carboxypropyl)sulfamoyl]-6,8-dihydroxypyren (9)

Zu einer Lösung des vorstehend synthetisierten bis-Boc-Derivats (8) (1,658 g, 2,09 mmol) in THF (200 mL) wurde auf einmal eine 2 N wässrige Lösung HCl hinzugegeben und für 24 h bei Raumtemperatur gerührt. Anschließend wurde unter reduziertem Druck zur Trockene eingedampft. ¹H NMR (300 MHz, D₂O): δ = 8,27 (s, 1 H), 7,15 (d, *J* = 9,0 Hz, 2 H), 6,96 (d, *J* = 9,0 Hz, 2 H), 5,83 (s, 1 H), 4,18 (t, *J* = 9.0 Hz, 2 H, 2 CHN), 2,81 (t, *J =* 9,0 Hz, 4 H, 2 C*H*₂N), 1,85 (m, 4 H, 2 CH₂).
Der Rückstand wurde in 80 ml Wasser aufgenommen und auf eine 4 cm x 40 cm Säule, gepackt mit saurem Dowex 50WX2-200, gegeben, mit 1,5 L Wasser gewaschen und anschließend mit 1,5 L 1N Ammoniak-Lösung eluiert. Die smaragdgrüne Lösung, welche unter reduziertem Druck bei 50°C eingedampft wurde, wurde über Phosphorpentoxid über Nacht getrocknet und als schwarz-braunes Pulver erhalten (1,113 g, 90%). Reinheit >95% (HPLC-MS). ¹H NMR (300 MHz, [D₆]-DMSO): δ = 9,0-8,0 (br. s, 8 H überlappend mit HO, NH und NH₂), 8,84 (s, 1 H), 8,55 (d, *J*= 9,0 Hz, 2 H), 8.46 (d, *J* = 9,0 Hz, 2 H), 7,29 (s, 1 H), 3,07 (m, 2 H, 2CHN), 2,87 (m, 4 H, 2 C*H*₂N), 1,74 (m, 4 H, 2 CH₂). HPLC-MS (ESI): *m*/*z* [M + H]⁺ berechnet für C₂₄H₂₇N₄O₁₀S₂ 595,1; gefunden 595,1. HRMS (ESI): *m*/*z* [M + Na]⁺ berechnet für C₂₄H₂₆N₄NaO₁₀S₂ 617,0988; gefunden 617,0100.

### Referenzen:

[D1] D. B. G. Williams, M. Lawton, J. Org. Chem. 2010, 75, 8351-8354
[D2] E. Koller, O. S. Wolfbeis, Austrian Patent AT 385 755 B, 1988**;** Chem Abstr. 1988, 111, 7091.
[D3] F. E. Cappuccio, J. T. Suri, D. B. Cordes, R. A. Wessling, B. Singaram, J. Fluoresc. 2004, 14, 521-533.
[D4] J. T. Suri, D. B. Cordes, F. E. Cappuccio, R. A. Wessling, B. Singaram, Angew. Chem. 2003, 115, 6037-6039.

## Patentansprüche

1. Fluoreszenzfarbstoff mit der folgenden Formel (I) 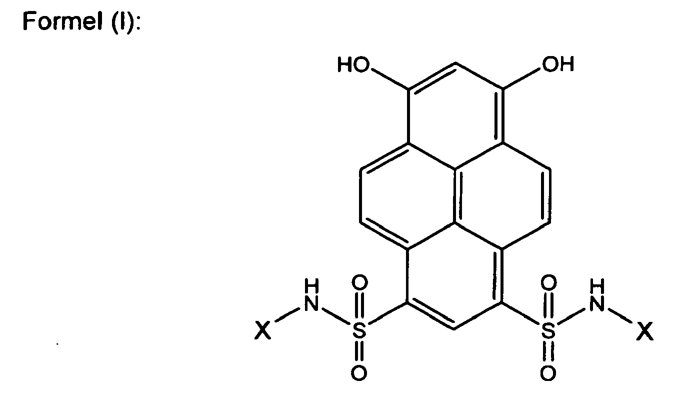 wobei X ausgewählt ist aus Wasserstoff, einer unsubstituierten oder substituierten C₁₋₂₀ Alkylgruppe, einer unsubstituierten oder substituierten C₁₋₂₀ Alkoxylgruppe, einer unsubstituierten oder substituierten C₁₋₂₀ Alkenylgruppe, einer unsubstituierten oder substituierten C₁₋₂₀ Alkinylgruppe, einer unsubstituierten oder substituierten C₁₋₂₀ Arylgruppe und einer unsubstituierten oder substituierten C₁₋₂₀ Heteroarylgruppe, wobei die Substituenten aus der Gruppe ausgewählt sind, umfassend Halogene, Hydroxyl, Carbonyl, Cyano, Thionyle, Aminogruppen, Amide und Carboxylgruppen, und deren Salze oder Kombinationen davon.

2. Fluoreszenzfarbstoff nach Anspruch 1 der folgenden Formel (II) 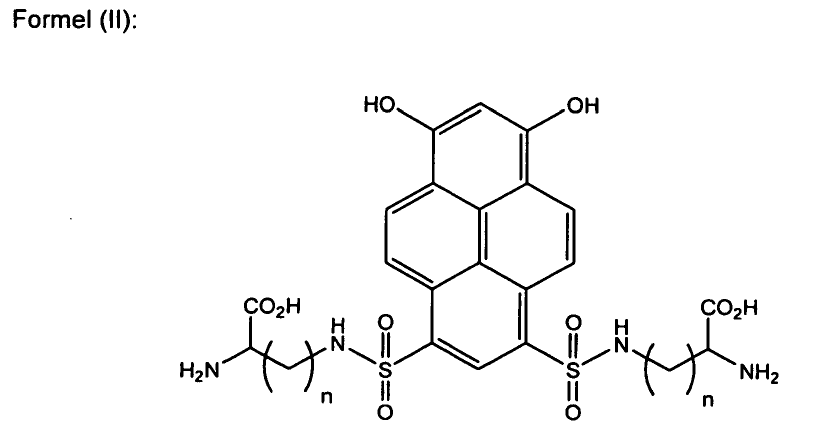 wobei n eine ganze Zahl von 1 bis 20 ist und die dadurch ausgedrückten Wiederholungseinheiten geradkettig oder verzweigtkettig sein können.

3. Fluoreszenzfarbstoff nach Anspruch 2, wobei n eine ganze Zahl von 2 bis 4 ist.

4. Fluoreszenzfarbstoff nach einem der Ansprüche 1 bis 3, wobei der Fluoreszenzfarbstoff zwei verschiedene Maxima der Fluoreszenzemission aufweist.

5. Fluoreszenzfarbstoff nach Anspruch 4, wobei mindestens eines der zwei verschiedenen Maxima der Fluoreszenzemission ein pH-abhängiges Intensitätsmaximum aufweist.

6. Fluoreszenzfarbstoff nach Anspruch 5, wobei das pH-abhängige Fluoreszenzmaximum in einem Bereich von > 420 nm liegt.

7. Verfahren zur Herstellung eines Fluoreszenzfarbstoffes mit der Formel (I), umfassend die Schritte des:
Umsetzens einer Verbindung der folgenden Formel (III) zu einer Verbindung der folgenden Formel (IV) und
Umsetzens der Verbindung mit der Formel (IV) mit einer Verbindung der Formel X-NHR, um den Fluoreszenzfarbstoff der Formel (I) zu erhalten, 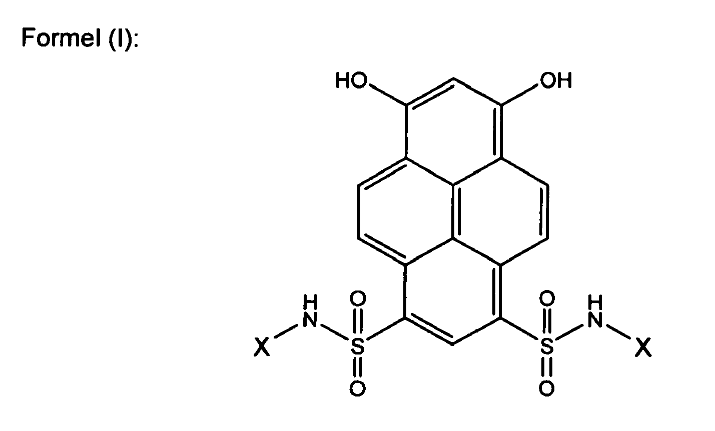 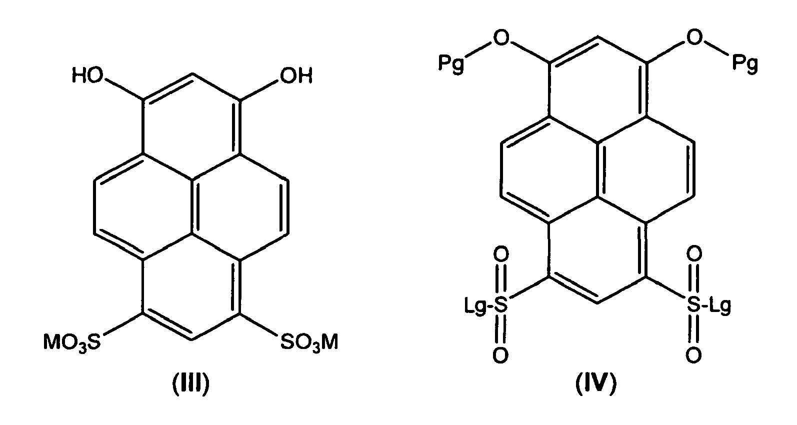
wobei X ausgewählt ist aus Wasserstoff, einer unsubstituierten oder substituierten C₁₋₂₀ Alkylgruppe, einer unsubstituierten oder substituierten C₁₋₂₀ Alkoxylgruppe, einer unsubstituierten oder substituierten C₁₋₂₀ Alkenylgruppe, einer unsubstituierten oder substituierten C₁₋₂₀ Alkinylgruppe, einer unsubstituierten oder substituierten C₁₋₂₀ Arylgruppe und einer unsubstituierten oder substituierten C₁₋₂₀ Heteroarylgruppe, wobei die Substituenten aus der Gruppe ausgewählt sind, umfassend Halogene, Hydroxyl, Carbonyl, Cyano, Thionyle, Aminogruppen, Amide und Carboxylgruppen, und deren Salze oder Kombinationen davon,
R ist ausgewählt aus Wasserstoff oder einer kationischen Gruppe,
M ist ausgewählt aus der Gruppe, bestehend aus Wasserstoff, Li, Na und K,
Pg stellt eine Schutzgruppe dar und
Lg ist ausgewählt aus der Gruppe, bestehend aus Triflat, Fluorosulfonat, Tosylat, Mesylat, Chlorid, Bromid und Iodid.

8. Verfahren nach Anspruch 7, wobei die Verbindung der Formel (III) das entsprechende Dinatriumsalz ist.

9. Verfahren nach Anspruch 7 oder 8, wobei als Verbindung X-NHR bei der Umsetzung mit der Verbindung der Formel (IV) eine Verbindung der folgenden Formel (V) eingesetzt wird, 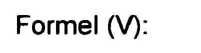 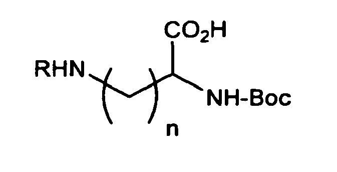 wobei R wie vorstehend definiert ist und n eine ganze Zahl von 1 bis 20 ist und die dadurch ausgedrückte Wiederholungseinheit geradkettig oder verzweigtkettig sein kann.

10. Optischer pH-Sensor, umfassend einen analytpermeablen Träger und den Fluoreszenzfarbstoff nach einem der Ansprüche 1 bis 6, welcher auf dem Träger immobilisiert ist.

11. Optischer pH-Sensor nach Anspruch 10, wobei die Dicke des Trägers von 50 µm bis 500 µm beträgt.

12. Optischer pH-Sensor nach Anspruch 10 oder 11, weiter umfassend mindestens eine Beschichtung, die auf mindestens einer Oberfläche des Trägers, auf welchem der Fluoreszenzfarbstoff immobilisiert ist, bereitgestellt ist.

13. Optischer pH-Sensor nach Anspruch 12, wobei die Beschichtung transparent ist.

14. Optischer pH-Sensor nach Anspruch 12 oder 13, wobei auf der Beschichtung eine Klebeschicht bereitgestellt ist.

15. Optischer pH-Sensor nach einem der Ansprüche 10 bis 14, wobei der dynamische Messbereich im Bereich von pH 4,0 bis pH 8,0 liegt.

## Claims

1. Fluorescent dye with the following Formula (I) 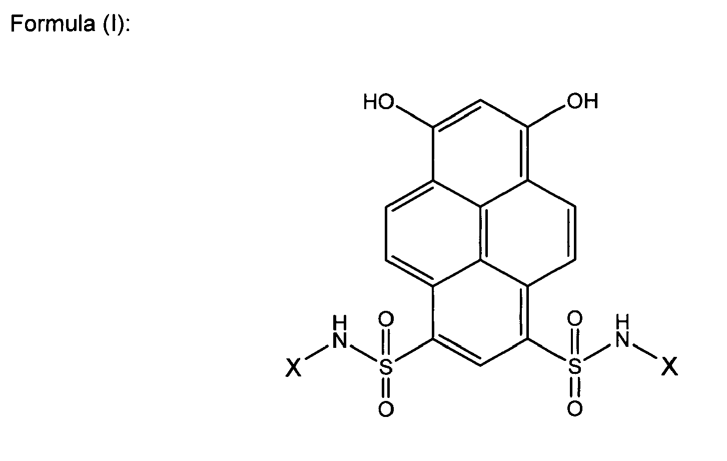 wherein X is selected from hydrogen, an unsubstituted or a substituted C₁₋₂₀ alkyl group, an unsubstituted or a substituted C₁₋₂₀ alkoxyl group, an unsubstituted or a substituted C₁₋₂₀ alkenyl group, an unsubstituted or a substituted C₁₋₂₀ alkinyl group, an unsubstituted or a substituted C₁₋₂₀ aryl group and an unsubstituted or a substituted C₁₋₂₀ heteroaryl group, wherein the substituents are selected from the group comprising halogens, hydroxyle, carbonyl, cyano, thionyls, amino groups, amides and carboxyl groups and the salts thereof or combinations.

2. Fluorescent dye according to claim 1 with the following Formula (II) 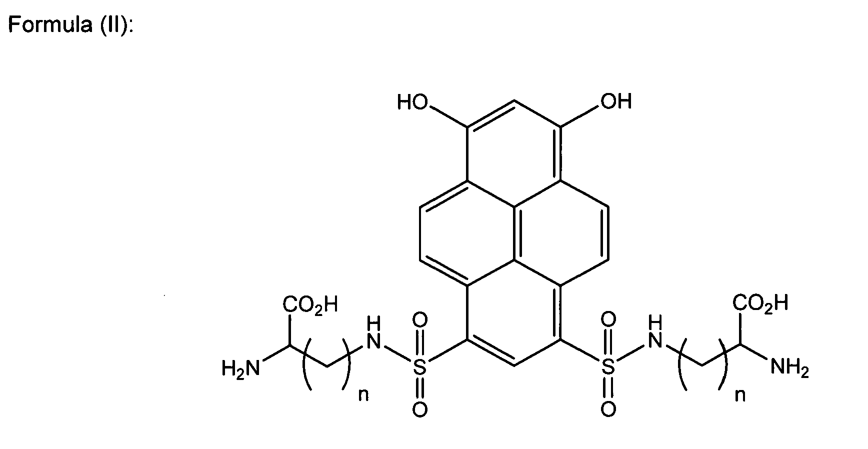 wherein n is an integral number from 1 to 20 and the thereby-expressed repetition units can be a straight chain or branched chain.

3. Fluorescent dye according to claim 2, wherein n is a integral number from 2 to 4.

4. Fluorescent dye according to any one of claims 1 to 3, wherein the fluorescent dye has two different maxima of the fluorescent emission.

5. Fluorescent dye according to claim 4, wherein at least one of the two different maxima of the fluorescent emission has a pH-dependent intensity maximum.

6. Fluorescent dye according to claim 5, wherein the pH-dependent fluorescent maximum lies in a range of > 420 nm.

7. Method of producing a fluorescent dye with the Formula (I), comprising the steps of:
converting a compound of the following Formula (III) into a compound of the following Formula (IV) and
converting the compound with the Formula (IV) by a compound of the formula X-NHR so as to obtain the fluorescent dye of Formula (I), 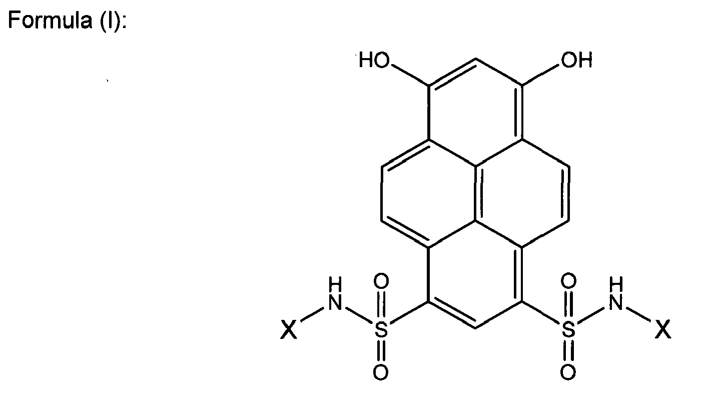 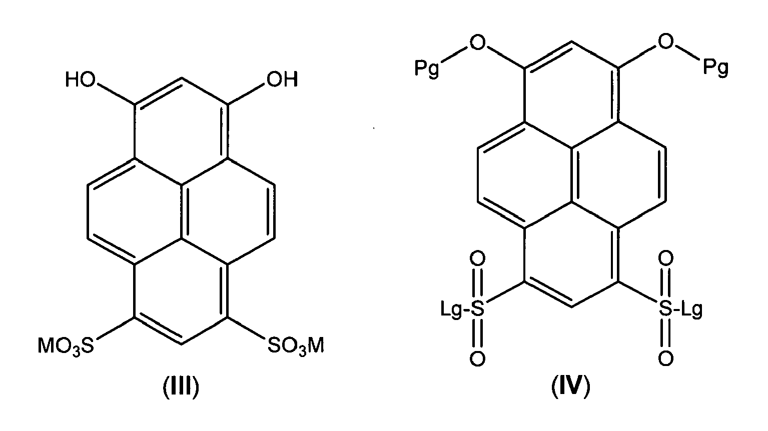
wherein X is selected from hydrogen, an unsubstituted or a substituted C₁₋₂₀ alkyl group, an unsubstituted or a substituted C₁₋₂₀ alkoxyl group, an unsubstituted or a substituted C₁₋₂₀ alkenyl group, an unsubstituted or a substituted C₁₋₂₀ alkinyl group, an unsubstituted or a substituted C₁₋₂₀ aryl group and an unsubstituted or a substituted C₁₋₂₀ heteroaryl group, wherein the substituents are selected from the group comprising halogens, hydroxyl, carbonyl, cyano, thionyls, amino groups, amides and carboxyl groups and the salts thereof or combinations,
R is selected from hydrogen or a cationic group,
M is selected from the group consisting of hydrogen, Li, Na and K,
Pg represents a protective group and
Lg is selected from the group consisting of triflate, fluorosulfonate, tosylate, mesylate, chloride, bromide and iodide.

8. Method according to claim 7, wherein the compound of the Formula (III) is the corresponding disodium salt.

9. Method according to claim 7 or 8, wherein a compound of the following Formula (V) is used as compound X-NHR in the conversion by the compound of the Formula (IV), 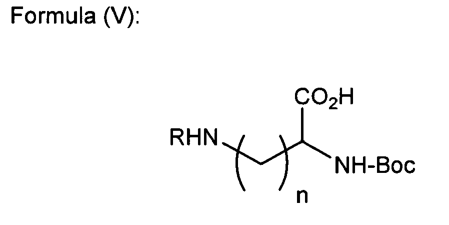 wherein R is as defined in the foregoing and n is an integral number from 1 to 20 and the thereby-expressed repetition unit can be a straight chain or branched chain.

10. Optical pH sensor, comprising an analyte-permeable support and the fluorescent dye according to any one of claims 1 to 6, which is immobilised on the carrier.

11. Optical pH sensor according to claim 10, wherein the thickness of the support is from 50 µm to 500 µm.

12. Optical pH sensor according to claim 10 or 11, further comprising at least one coating which is provided on at least one surface of the support, on which the fluorescent dye is immobilised.

13. Optical pH sensor according to claim 12, wherein the coating is transparent.

14. Optical pH sensor according to claim 12 or 13, wherein an adhesive layer is provided on the coating.

15. Optical pH sensor according to any one of claims 10 to 14, wherein the dynamic measuring range lies in the range of pH 4.0 to pH 8.0.

## Revendications

1. Colorant fluorescent avec la formule (I) suivante 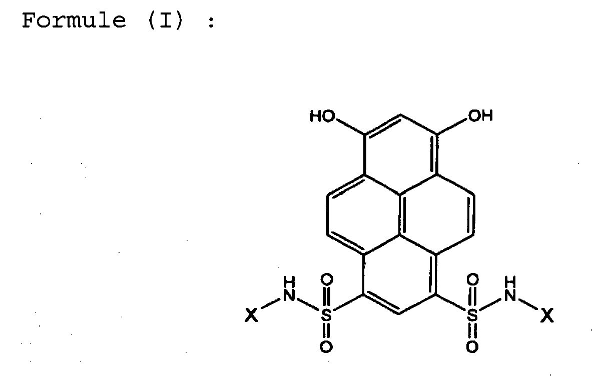 où X est choisi parmi un atome d'hydrogène, un groupe alkyle en C₁₋₂₀ non substitué ou substitué, un groupe alcoxyle en C₁₋₂₀ non substitué ou substitué, un groupe alcényle en C1-20 non substitué ou substitué, un groupe alcynyle en C₁₋₂₀ non substitué ou substitué, un groupe aryle en C₁₋₂₀ non substitué ou substitué et un groupe hétéroaryle en C₁₋₂₀ non substitué ou substitué, les substituants étant choisis dans le groupe comprenant des atomes d'halogènes, un groupe hydroxyle, un groupe carbonyle, un groupe cyano, un groupe thionyle, des groupes amine, des groupes amide et carboxyle, et leurs sels ou combinaisons.

2. Colorant fluorescent selon la revendication 1 de la formule (II) suivante, 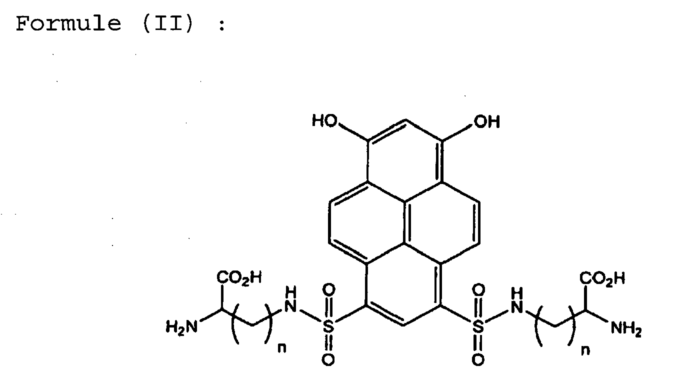 où n est un nombre entier de 1 à 20 et où les unités de répétition ainsi matérialisées peuvent être en chaîne linéaire ou en chaine ramifiée.

3. Colorant fluorescent selon la revendication 2, où n est un nombre entier entre 2 et 4.

4. Colorant fluorescent selon l'une des revendications 1 à 3, où le colorant fluorescent présente deux valeurs maximales d'émission de fluorescence différentes.

5. Colorant fluorescent selon la revendication 4, où au moins une des deux valeurs maximales d'émission de fluorescence présente un maximum d'intensité dépendant du pH.

6. Colorant fluorescent selon la revendication 5, où le maximum de fluorescence dépendant du pH se situe dans un domaine > à 420 nm.

7. Procédé de fabrication d'un colorant fluorescent avec la formule (I),
comprenant les étapes de :
réaction d'un composé de la formule (III) suivante avec un composé de la formule (IV) suivante, et
réaction du composé de la formule (IV) avec le composé de la formule X-NHR, afin d'obtenir le colorant fluorescent de la formule (I), 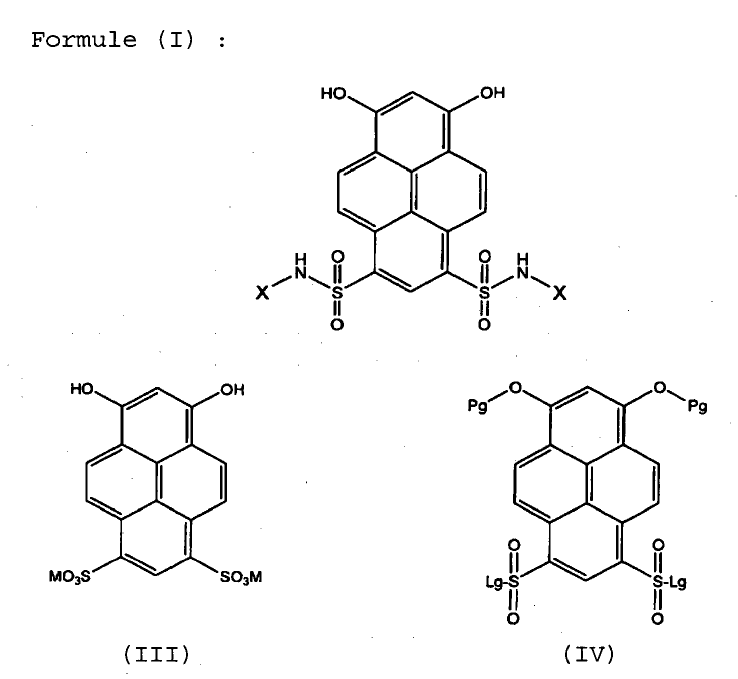
où X est choisi parmi un atome d'hydrogène, un groupe alkyle en C₁₋₂₀ non substitué ou substitué, un groupe alcoxyle en C₁₋₂₀ non substitué ou substitué, un groupe alcényle en C₁₋₂₀ non substitué ou substitué, un groupe alcynyle en C₁₋₂₀ non substitué ou substitué, un groupe aryle en C₁₋₂₀ non substitué ou substitué et un groupe hétéroaryle en C₁₋₂₀ non substitué ou substitué, les substituants étant choisis dans le groupe comprenant des atomes d'halogènes, un groupe hydroxyle, un groupe carbonyle, un groupe cyano, un groupe thionyle, des groupes amine, des groupes amide et carboxyle, et leurs sels ou combinaisons,
R est choisi parmi un atome d'hydrogène ou un groupe cationique,
M est choisi dans le groupe constitué par un atome d'hydrogène, un atome de Li, un atome de Na et un atome de K,
Pg représente un groupe protecteur, et
Lg est choisi dans le groupe constitué par un groupe triflate, un groupe fluorosulfonate, un groupe tosylate, un groupe mésylate, un groupe chlorure, un groupe bromure et un groupe iodure.

8. Procédé selon la revendication 7, où le composé de la formule (III) est le sel disodique correspondant.

9. Procédé selon les revendications 7 ou 8, où, en tant que composé X-NHR lors de la réaction avec le composé de la formule (IV), on emploie un composé de la formule (V) suivante, 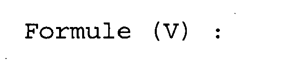 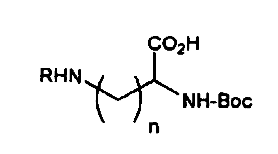 où R est défini comme précédemment et n est un nombre entier de 1 à 20 et où l'unité de répétition ainsi matérialisée peut être en chaîne linéaire ou en chaîne ramifiée.

10. Capteur de pH optique, comprenant un support d'analyte perméable et le colorant fluorescent selon l'une des revendications 1 à 6, lequel est immobilisé sur un support.

11. Capteur de pH optique selon la revendication 10, où l'épaisseur du support se situe entre 50 µm et 500 µm.

12. Capteur de pH optique selon les revendications 10 ou 11, comprenant en outre au moins un revêtement qui est déposé sur au moins une surface du support sur laquelle le colorant fluorescent est immobilisé.

13. Capteur de pH optique selon la revendication 12, où le revêtement est transparent.

14. Capteur de pH optique selon les revendications 12 ou 13, où une couche d'adhésif est déposée sur le revêtement.

15. Capteur de pH optique selon l'une des revendications 10 à 14, où le domaine de mesure dynamique se situe dans le domaine de pH 4,0 à pH 8,0.
